(19) Europäisches Patentamt
European Patent Office
Office européen des brevets

(11) Publication number: **0 406 748 B1**

## EUROPEAN PATENT SPECIFICATION

(45) Date of publication of patent specification: **28.09.94**

(51) Int. Cl.5: **C07C 17/34**, C07C 17/00, C07C 21/18, B01J 37/26

(21) Application number: **90112561.7**

(22) Date of filing: **02.07.90**

(54) **Dehydrofluorination and dehydrogenation of fluorinated alkanes.**

(30) Priority: **06.07.89 US 376084**
**08.06.90 US 535274**

(43) Date of publication of application:
**09.01.91 Bulletin 91/02**

(45) Publication of the grant of the patent:
**28.09.94 Bulletin 94/39**

(84) Designated Contracting States:
**BE DE FR GB IT NL**

(56) References cited:
**EP-A- 0 234 002**
**FR-A- 2 152 990**
**US-A- 3 432 562**
**US-A- 3 456 025**
**US-A- 3 607 955**

(73) Proprietor: **ATOCHEM NORTH AMERICA, INC.**
**Three Parkway**
**Philadelphia, Pennsylvania 19102 (US)**

(72) Inventor: **Bolmer, Michael Sheppard**
**5033 Cold Springs Drive**
**Collegeville, Pennsylvania 19426 (US)**
Inventor: **Elsheikh, Maher Yousef**
**784 North Wayne Avenue**
**Wayne, Pennsylvania 19087 (US)**

(74) Representative: **Kraus, Walter, Dr. et al**
**Patentanwälte Kraus, Weisert & Partner**
**Thomas-Wimmer-Ring 15**
**D-80539 München (DE)**

**Description**

The invention relates to the dehydrofluorination and dehydrogenation of fluorinated alkanes. In particular, the invention relates to the dehydrofluorination of trifluoroalkanes, and the dehydrogenation of 1,1-difluoroethane.

**Background of the Invention**

The dehydrofluorination of polyfluoroalkanes to fluoroalkenes in the presence of chromium salts is known. U.S. Patent 2,461,253 discloses the use of $CrF_3$ or $CrF_3$ on activated carbon for such purposes. Japanese Patent Application 54/130507 describes the production of vinylidene fluoride by dehydrofluorination of 1,1,1-trifluoroethane in the presence of an inorganic chromium (III) compound. European Patent 234,002 discloses the conversion of 1,1,1-trifluoroalkanes to 1,1-difluoroalkenes in the presence of a catalyst bed of an inorganic chromium oxyfluoride at a reaction temperature of above 430°C. The catalyst bed is periodically regenerated with hot oxygen when the conversion rate falls.

The pyrolysis of polyfluoroalkanes in the presence or absence of a catalyst is described in U.S. Patent 2,480,560. Catalysts include heavy metal halides, such as heavy metal halides of iron, copper and nickel, and halides of alkaline earth metals, such as $BaCl_2$ and $CaCl_2$.

Fluorinated alumina catalysts have also been employed in the dehydrofluorination of polyfluoroalkanes. U.S. Patents 3,456,025 and 3,432,562 disclose the dehydrofluorination of trifluoroalkanes utilizing a fluorinated alumina catalyst prepared by the vapor phase reaction of hydrogen fluoride with $\eta$- or $\gamma$-alumina. In this manner, 1,1,1-trifluoroethane was dehydrofluorinated to vinylidene fluoride with 21% conversion at 482°C. See Pat. 3,456,025. Pat. 3,432,562 reports the use of ammonium bifluoride supported on $AlF_3$ and $CrF_3$ to dehydrofluorinate 1,1,2-trifluoroethane into a mixture of cis and trans 1,2-difluoroethylene at 427°C. Pat. 3,432,562 further discloses that metals such as zinc, chromium, cobalt, silver, copper, vanadium, iron, nickel, lead, antimony and tin may be used as catalysts for the dehydrofluorination of trifluoroalkanes to difluoroalkenes, at temperatures between 200°C and 538°C, and pressures ranging from 0 to 100 PSIG. Neither of Patents 3,456,025 or 3,432,562 report data on catalyst operational lifetime, or catalyst regenerability.

U.S. Patent 3,607,955 describes a hydrofluorination and dehydrofluorination catalyst prepared from the reaction of alumina and hydrogen fluoride at elevated temperatures.

The dehydrohalogenation of 1,1,1-trifluorohaloalkanes to form gem-difluoroalkenes in the presence of a low surface area alumina catalyst is described in U.S. Patent 3,444,251.

U.S. Patent 2,442,993 describes the pyrolytic elimination of hydrogen fluoride from polyfluoroalkanes at 250-700°C in the presence of 0.1%-10% $O_2$.

Walker et al., **J. Org. Chem.** 30, 3284-5 (1965) describe the dehydrohalogenation of 1,1,1-trifluoroethane and 1,1,1-difluorochloroethane in the presence of a variety of catalysts including alumina, at 200-400°C. Dehydrochlorination and dehydrofluorination were observed, according to the catalyst and reaction conditions employed.

Vinylidene fluoride is typically produced by dehydrochlorination of 1-chloro-1,1-difluoroethane. However, 1-chloro-1,1-difluoroethane is a relatively costly intermediate. 1,1-Difluoroethane, on the other hand, is relatively easily provided from the reaction of vinylidene chloride and hydrogen fluoride, such as described in U.S. Patent 3,978,145.

Vinylidene fluoride has been produced directly from 1,1-difluoroethane by dehydrogenation utilizing ultraviolet irradiation. Carmichael et al., **J. Phys. Chem.** 78(22), 2183-6 (1974). Conversion of 1,1-difluoroethane was very low, only 100 parts per million.

U.S. Patents 2,722,558 and 2,723,296 disclose a multi-step process for production of vinylidene fluoride from 1,1-difluoroethane. Chlorine gas is utilized as co-reactant to convert 1,1-difluoroethane to 1-chloro-1,1-difluoroethane. The latter is thereafter dehydrochlorinated into vinylidene fluoride.

An improved process for dehydrofluorinating fluorinated alkanes, including mono-, di-, tri- and tetrafluoroalkanes, is possible utilizing a catalyst which includes $\gamma$-alumina. The process is characterized by conversion of the fluorinated alkane to the desired unsaturated dehydrofluorination product, and an extended catalyst lifetime. The same catalyst is useful for catalyzing the dehydrogenation of 1,1-difluoroethane, thereby enabling the direct production of vinylidene fluoride from 1,1-difluoroethane in a single step.

## Summary of the Invention

A process for dehydrofluorinating a fluorinated alkane of the formula $R_1R_2CHCFR_3R_4$ is provided, wherein each of $R_1$, $R_2$, $R_3$ and $R_4$ are independently selected from the group of hydrogen, fluorine, methyl, ethyl, propyl, t-butyl and phenyl. The fluorinated alkane and a co-reactant gas containing oxygen, carbon dioxide or mixtures thereof is flowed over a catalyst bed including $\gamma$-fluorided alumina at a reaction temperature of from 200°C to 700°C, the catalyst having been prepared by the steps of:

(a) flowing air over a catalyst bed including $\gamma$-alumina for a contact time of from 5 seconds to 150 seconds, at a temperature of from 600°C to 750°C, and

(b) thereafter flowing hydrogen fluoride over the catalyst at elevated temperature to convert the $\gamma$-alumina to fluorided $\gamma$-alumina.

According to a preferred embodiment of the invention, the process is directed to the production of 1,1-difluoroalkenes of the formula $R_1CH=CF_2$ from 1,1,1-trifluoroalkanes of the formula $R_1CH_2CF_3$, where $R_1$ is defined as above.

A process for producing vinylidene fluoride from 1,1-difluoroethane is also provided. 1,1-Difluoroethane and a co-reactant gas containing oxygen, carbon dioxide or mixtures thereof is flowed over a catalyst bed including $\gamma$-fluorided alumina at a reaction temperature of from 300°C to 700°C, the catalyst having been prepared by steps (a) and (b), above. Vinylidene fluoride is recovered from the resulting mixture comprising vinyl fluoride and vinylidene fluoride.

## Detailed Description of the Invention

Dehydrofluorination of fluorinated alkanes, i.e., alkanes wherein one or more hydrogen atoms are substituted by fluorine atoms, is achieved according to the process of the invention in a substantially uninterrupted continuous basis, without the need for periodic catalyst regeneration. Unlike certain prior art dehydrofluorination processes, e.g. the process described in European Patent 234,002, the catalyst described herein is capable of operating over extended periods of time, in some cases up to one month or longer, without regeneration.

The catalyst is prepared from $\gamma$-alumina. The alumina may be used alone in the catalyst preparation, or it may be combined with a trivalent chromium or iron compound, preferably, $CrF_3$ or $FeF_3$. Other salts of chromium (III) or iron (III), or oxides thereof, may be utilized. Trivalent salts useful for this purpose include, for example, the nitrates, sulfates and halides of chromium (III) and iron (III).

The trivalent chromium or iron compound may be incorporated into the $\gamma$-alumina in any convenient manner. Preferably, the alumina is impregnated with a solution of the metal-containing compound. Thus, for example, the catalyst may be prepared, for example, from $Cr_2O_3$ supported on $\gamma$-alumina, which is thereafter activated as hereinafter described, such that a substantial portion of the $Cr_2O_3$ and $\gamma$-alumina comprising said catalyst is converted to corresponding fluorine-containing compounds, principally $CrF_3$ and $AlF_3$.

Preferably, the catalyst bed is prepared from $\gamma$-alumina alone. When activated, the $\gamma$-alumina forms fluorided $\gamma$-alumina. As used herein, "fluorided $\gamma$-alumina" means $\gamma$-alumina (alumina also being known by its empirical formula $Al_2O_3$), a substantial portion of which has been converted to fluorine-containing compounds of aluminum by the catalyst activation process essentially described herein. Without wishing to be bound by any theory, it is believed that the principal catalytic species thus formed is $AlF_3$.

The manner in which the $\gamma$-alumina catalyst bed is activated is critical to dehydrofluorination performance. The extent to which the fluorinated alkane is converted to the corresponding dehydrofluorination product is dependent, to some extent, on the catalyst activation. Gamma-alumina, alone or in combination with one or more of the aforementioned trivalent chromium or iron compounds, is packed into a suitable reactor vessel to form a catalyst bed. The catalyst is then activated by flowing air. The contact time of the air with the catalyst is from 5 seconds to 150 seconds, preferably from 20 seconds to 120 seconds. By "contact time" in this context is meant the catalyst bed volume, divided by the flow rate of gas (in this case air) contacted with the catalyst bed. The temperature of the air is from 600°C to 750°C preferably from 650°C to 700°C. At temperatures higher than 750°C, the catalyst may begin to sinter. Catalyst sintering is undesirable since it may induce a phase change in the alumina from the $\gamma$ phase, resulting in a loss in catalyst efficiency. Air temperatures below 600°C are generally insufficient to induce substantial activation of $\gamma$-alumina. Optimally, air activation of the catalyst bed is achieved by employing an air flow over the $\gamma$-alumina-containing bed at a rate of about 20 cm$^3$/minute for 18 hours at about 650°C.

Following air activation treatment, the catalyst bed is subjected to activation by hydrogen fluoride. The amount of hydrogen fluoride consumed in the activation process depends upon the amount of $\gamma$-alumina in the bed. Hydrogen fluoride is flowed over the catalyst at, for example, a temperature of from 500°C to 600°C. The feed rate of hydrogen fluoride has little or no impact on catalyst activation. However, about eight hours is the recommended time for the hydrogen fluoride activation step.

Hydrogen fluoride activation of the catalyst is optionally, but preferably, carried out in the presence of an inert carrier gas such as nitrogen. Although nitrogen is the preferred carrier gas, any other gas, which is inert toward the catalysts, reactants, and products of the invention under the reaction conditions employed, may be used. Such inert gases include, in addition to nitrogen, helium, neon, argon, and the like. The use of a carrier gas is desirable in order to dissipate the heat generated by the highly exothermic conversion of $\gamma$-alumina to fluorided $\gamma$-alumina, thereby avoiding catalyst sintering. Carbon dioxide is disclosed herein as a co-reactant gas in the dehydrofluorination process of the invention because it favorably interacts with the hydrofluorination catalyst to prolong catalyst lifetime by continuously removing carbonaceous material from the catalyst surface. Carbon dioxide is further useful as a carrier gas. It is effective in dissipating heat and protecting the catalyst from sintering. Typically, from 100 to 500 volumes of the carrier gas, per 100 volumes of the hydrogen fluoride gas flowed over the catalyst, may be utilized.

Following processing of the catalyst substantially as described above, the fluorinated alkane, e.g. 1,1,1-trifluoroethane, is passed through the reactor and over the catalyst bed with a co-reactant gas containing oxygen, carbon dioxide, or mixtures thereof. We have found that the effective catalyst lifetime of fluorided $\gamma$-alumina-containing catalysts may be significantly extended by combining the fluorinated alkane with a small amount of co-reactant gas. The co-reactant gas may comprise substantially pure oxygen or substantially pure carbon dioxide, or mixtures thereof. Alternatively, the co-reactant gas may comprise a mixture of oxygen and/or carbon dioxide in admixture with other gases which do not interfere with the dehydrofluorination reaction. For example, the co-reactant gas may advantageously comprise atmospheric air, which principally contains nitrogen, oxygen and carbon dioxide.

For dehydrofluorination, the reaction temperature in the reactor vessel is from 200°C to 700°C, preferably from 400°C to 700°C, most preferably from 500°C to 650°C. For dehydrogenation of 1,1-difluoroethane, the reaction temperature in the reactor vessel is from 300°C to 700°C, preferably from 400°C to 550°C.

Where the co-reactant gas is an oxygen-containing gas, i.e., substantially pure oxygen, air or a synthetic mixture of oxygen with mostly inert gases, from about 2 to about 10 volumes of oxygen is combined with each 100 volumes of the fluorinated alkane and passed over the catalyst to achieve dehydrofluorination. Thus, for example, where the volume of the fluorinated alkane is 100 cm$^3$, from 2 to 10 cm$^3$ of oxygen are utilized. Above 10% oxygen, sintering of the catalyst may occur. Below 2% oxygen, the catalyst may become deactivated, thus requiring periodic interruption of the dehydrofluorination process to permit catalyst reactivation. Preferably, from 2 to 6 volumes of oxygen are combined with each 100 volumes of the fluorinated alkane.

Where the co-reactant gas comprises carbon dioxide or a carbon dioxide-containing gas, the fluorinated alkane is combined therewith in the amount of from 50 to 400 volumes of carbon dioxide, per 100 volumes of fluorinated alkane and passed over the catalyst bed. Above about 400% carbon dioxide, the fluorinated alkane becomes too dilute, and dehydrofluorination efficiency is lost. Below about 50% carbon dioxide, the catalyst may become deactivated, requiring periodic regeneration.

The fluorinated alkane and co-reactant gas may be optionally combined with an inert carrier gas such as nitrogen, helium, argon, or the like. Nitrogen is preferred. The carrier gas may be combined with the other feed gases in an amount of from zero to about 300 volumes, per 100 volumes of the fluorinated alkane.

For dehydrogenation of 1,1-difluoroethane to vinylidene fluoride, the co-reactant gas is advantageously utilized in a mole ratio with 1,1-difluoroethane of from 0.04:1 to 10:1, preferably from 0.5:1 to 4:1.

For dehydrofluorination of fluorinated alkanes, the combined flow rates of the fluorinated alkane, co-reactant gas and carrier gas over the catalyst bed are advantageously selected to provide a total contact time of all gases with the catalyst, of from 1 second to 100 seconds, preferably from 5 seconds to 50 seconds. Longer contact times could be employed, but are not necessary. For dehydrogenating 1,1-difluoroethane to vinylidene fluoride, the flow rates of the gases are advantageously selected to provide a total contact time of all gases with the catalyst of from 1 second to 60 seconds, preferably from 5 seconds to 20 seconds. By "contact time" in this context is meant the catalyst bed volume, divided by the sum of the flow rates of the fluorinated alkane, co-reactant gas and carrier gas contacted with the catalyst bed.

The fluorinated alkanes which may be converted to fluorinated alkenes (or alkenes, in the case of dehydro fluorination of monofluoroalkanes), contain at least two carbon atoms, with at least one fluorine atom attached to one of the carbon atoms, and at least one hydrogen atom attached to an adjacent carbon atom. Thus, possible reactants which may be converted to their corresponding unsaturated dehydrofluorination products include the fluorinated alkanes identified below. The list is intended to be illustrative only.

4

Some products may appear as mixtures of fluoroalkene isomers. Thus, while one possible dehydrofluorination product is stated for each starting fluoroalkene, it will be appreciated that the actual dehydrofluorination product may comprise a mixture of isomers having the same molecular weight.

| Monofluoroalkanes | Dehydrofluorination Product |
|---|---|
| fluoroethane | ethylene |
| fluoropropane | propene |

| gem-Difluoroalkanes ($R_3$=F) | A Possible Dehydrofluorination Product |
|---|---|
| 1,1-difluoroethane | fluoroethylene |
| 1,1-difluoropropane | 1-fluoro-1-propene |
| 2,2-difluoropropane | 2-fluoro-1-propene |
| 1,1-difluorobutane | 1-fluoro-1-butene |
| 1,1-difluoro-3-methylbutane | 1-fluoro-3-methyl-1-butene |

| vic-Difluoroalkanes ($R_2$=F) | A Possible Dehydrofluorination Product |
|---|---|
| 1,2-difluoroethane | fluoroethylene |

| Trifluoroalkanes ($R_2$,$R_3$=F) | A Possible Dehydrofluorination Product |
|---|---|
| 1,1,2-trifluoroethane | 1,1-difluoroethylene |
| 1,1,2-trifluoropropane | 1,2-difluoro-1-propene |
| 1,2,2-trifluoropropane | 1,2-difluoro-2-propene |
| 2,2,3-trifluorobutane | 2,3-difluoro-2-butene |
| 1,1,2-trifluorobutane | 1,2-difluoro-1-butene |
| 1,1,2-trifluoropentane | 1,2-difluoro-1-pentene |

| 1,1,1-Trifluoroalkanes ($R_3$,$R_4$=F) | A Possible Dehydrofluorination Product |
|---|---|
| 1,1,1-trifluoroethane | 1,1-difluoroethylene |
| 1,1,1-trifluoropropane | 1,1-difluoro-1-propene |
| 1,1,1-trifluorobutane | 1,1-difluoro-1-butene |
| 1,1,1-trifluoropentane | 1,1-difluoro-1-pentene |
| 1,1,1-trifluoro-2-methylpropane | 1,1-difluoro-2-methyl-1-propene |
| 1,1,1-trifluoro-3-methylbutane | 1,1-difluoro-3-methyl-1-butene |
| 1,1,1-trifluoro-2-ethylbutane | 1,1-difluoro-2-ethyl-1-butene |
| 1,1,1-trifluoro-2-ethylpentane | 1,1-difluoro-2-ethyl-1-pentene |
| 1,1,1-trifluoro-3,3-dimethylbutane | 1,1-difluoro-3,3-dimethyl-1-butene |
| 1,1,1-trifluoro-2,3,3-trimethylbutane | 1,1-difluoro-2,3,3-trimethyl-1-butene |

| Tetrafluoroalkanes ($R_2$,$R_3$,$R_4$=F) | A Possible Dehydrofluorination Product |
|---|---|
| 1,1,1,2-tetrafluoroethane | 1,1,2-trifluoroethylene |
| 1,1,1,2-tetrafluoropropane | 1,1,2-trifluoro-1-propene |
| 1,1,1,2-tetrafluorobutane | 1,1,2-trifluoro-1-butene |
| 1,1,1-2-tetrafluoro-3,3-dimethylbutane | 1,1,2-trifluoro-3,3-dimethyl-1-butene |

5

The dehydrofluorination process is particularly useful for converting 1,1,1-trifluoroethane to vinylidene fluoride. 1,1,1-Trifluoroethane is a waste product of the hydrofluorination of methyl chloroform to 1-chloro-1,1-difluoroethane. According to the present invention, 1,1,1-trifluoroethane is readily converted to two useful products, vinylidene fluoride and hydrogen fluoride.

The process is further particularly useful for converting 1,1-difluoroethane to the dehydrofluorination product vinyl fluoride, and/or the dehydrogenation product vinylidene fluoride. At a reaction temperature of at least 300°C, the dehydrogenation product, vinylidene fluoride, is generated in small amounts. Selectivity for the minor product, vinylidene fluoride, increases with increasing temperature.

Vinylidene fluoride may be advantageously recovered from the reaction product by separating it from the major product, vinyl fluoride, by distillation. Vinylidene fluoride and vinyl fluoride have normal boiling points of -86°C and -72°C, respectively.

Separation may be advantageously accomplished by scrubbing hydrogen fluoride and $CO_2$ from the reaction product, removing air and water therefrom, and then finally feeding the reaction product to a distillation column. Vinylidene fluoride may be taken off as the distillation overhead product at a distillation column top temperature of, e.g., -3°C. Vinyl fluoride is removed as the column bottom product, at a column bottom temperature of, e.g., 14°C. The column is advantageously equipped with a condenser, cooled with, for instance, circulating refrigerated brine. The distillation column may be operated at a pressure below 300 psi, provided the condenser is adequately refrigerated to a lower temperature. The optimum balance between compressor cost and refrigeration cost may be readily ascertained by those skilled in the art by routine experimentation. Unreacted 1,1-difluoroethane may be distilled from the vinyl fluoride product and recycled back to the reactor.

The process of the invention is illustrated in the following non-limiting examples, wherein percentages of reactor feed gases are by volume, and percentages of fluorinated alkane conversion to dehydrofluorination product are by mole.

## Example 1

Gamma-alumina (Alpha Chemical Co., #89372, 79.7 g) was loaded into a Hastelloy C tubular reactor (1″ inside diameter x 13″ length). The reactor was gradually heated to 650°C with the continuous feed of air from a valve at the reactor top at the rate of 20 cm³/min. The temperature was maintained at 650°C with the same air flow rate for 18 hours. The catalyst was thereafter activated at 550°C with hydrogen fluoride fed at the rate of 0.06 g/min. for 6 hours from a valve at the reactor top, with nitrogen as a carrier gas at the rate of 20 cm³/min. to form fluorided $\gamma$-alumina. Following activation, 1,1,1-trifluoroethane, nitrogen and oxygen were fed to the reactor continuously in a mixture of 75% nitrogen, 23.4% 1,1,1-trifluoroethane and 1.6% oxygen, by volume. The reaction temperature was 500°C. The reaction products were removed at the bottom of the reactor and passed up to a scrubbing tower, counter-current to a liquid alkaline stream of 1-5 N aqueous KOH, in order to remove hydrogen fluoride. The organic product was then passed through a drying tower packed with a drying agent (anhydrous calcium sulfate). The conversion was periodically checked by passing product automatically to a gas chromatograph equipped with an electronic integrator. The mass balance was evaluated by passing the outlet gas from the gas chromatograph through a wet test meter. Gas chromatographic analysis indicated a constant 18% conversion of 1,1,1-trifluoroethane to vinylidene fluoride over 20 hours of continuous operation, with 100% selectivity for vinylidene fluoride.

## Example 2

The procedure of Example 1 was repeated, except that the reaction temperature was increased to 550°C. A substantially constant 42% conversion of 1,1,1-trifluoroethane to vinylidene fluoride was maintained for 7.5 hours, with 100% selectivity for vinylidene fluoride.

## Example 3

The procedure of Example 1 was followed except that the reaction temperature was increased to 550°C, and the feed utilized was 6.25% oxygen and 93.75% 1,1,1-trifluoroethane, by volume. An initial 45% rate of 1,1,1-trifluoroethane conversion decreased only to 40%, even after 240 hours of continuous catalyst operation. Selectivity for vinylidene fluoride was 100%.

6

## Example 4

A $CrF_3/\gamma$-alumina catalyst was prepared as follows. $CrF_3 \cdot 3H_2O$ (44 g) was dissolved in 220 ml of water using a Soxhlet extractor for continuous dissolution. To the resulting hot, dark green solution was added 200 g of $\gamma$-alumina (Alpha Chemical Co., 2-3 mm pellets). The resulting mixture was left standing for 60 hours at room temperature. The light green coated pellets were collected by filtration and dried in air, and then 85 g of the catalyst was loaded into the same reactor utilized in Example 1. The catalyst was activated at 650°C with 20 $cm^3$/min. of air for 18 hours, followed by HF (0.06 g/min.) and nitrogen (20 $cm^3$/min.) at 550°C for 24 hours. 1,1,1-Trifluoroethane and $CO_2$ were continuously fed to the reactor (75% $CO_2$ and 25% 1,1,1-trifluoroethane, by volume) at a reaction temperature of 400°C. The initial conversion of 1,1,1-trifluoroethane was 27%, which dropped to 18% over the course of 13 hours of continuation reactor operation. Selectivity for vinylidene fluoride was 100%.

## Example 5

Example 4 was repeated except that the reaction temperature was increased to 500°C. The conversion of 1,1,1-trifluoroethane climbed from an initial value of 32% to reach 42% after 45 hours of continuous operation. Selectivity for vinylidene fluoride was 100%.

## Example 6

The procedure of Example 4 was repeated except that the reaction temperature was increased to 550°C. 1,1,1-Trifluoroethane conversion dropped from an initial value of 65% to a final value of 56% over the course of 33 hours of continuous reactor operation. Selectivity for vinylidene fluoride was 100%.

## Example 7

A 8.5% $CrF_3/\gamma$-alumina catalyst was prepared as follows. $CrF_3 \cdot 3H_2O$ (22 g) was dissolved in 210 ml of water using a Soxhlet extractor for continuous dissolution. To the resulting hot, dark green solution was added 200 g of $\gamma$-alumina (Alpha Chemical Co., 2-3 mm pellets). The resulting mixture was left standing for 60 hours at room temperature. The green extrudate was air dried, then 93.5 g of the solid was loaded into the same reactor as used in Example 1. The catalyst was activated with air at 650°C using 20 $cm^3$/min. air for 18 hours, followed by hydrogen fluoride activation. The latter was achieved with a mixture of hydrogen fluoride and nitrogen, fed at rates of 0.06 g/min. and 20 $cm^3$/min., respectively, for 24 hours. A mixture of 75% $CO_2$ and 25% 1,1,1-trifluoroethane, by volume, was then continuously fed to the reactor at a reaction temperature of 500°C. Conversion of 1,1,1-trifluoroethane to vinylidene fluoride was initially measured at 38.5%, increasing to 42.7% after 35 hours of continuous reactor operation. Selectivity for vinylidene fluoride was 100%.

## Example 8

Example 5 was repeated except that the reaction temperature was increased to 550°C. Conversion of 1,1,1-trifluoroethane to vinylidene fluoride, initially measured at 52%, decreased to 40% over 20 hours of continuous operation. Selectivity for vinylidene fluoride was 100%.

## Example 9

The procedure of Example 7 was repeated except that the reactor feed was 74% nitrogen, 24.4% 1,1,1-trifluoroethane and 1.6% oxygen, by volume. A steady 38% conversion of 1,1,1-trifluoroethane was maintained over 48 hours of continuous operation. Selectivity for vinylidene fluoride was 100%.

## Example 10

The procedure of Example 9 was repeated except that the reaction temperature was increased to 550°C. An initial 48% conversion of 1,1,1-trifluoroethane to vinylidene fluoride increased to 57% over 24 hours of continuous operation. Selectivity for vinylidene fluoride was 100%

**Comparative Examples 11-13**

An 8.5 wt.% $CrF_3/AlF_3$ dehydrofluorination catalyst was prepared as follows. $CrF_3 \cdot 3H_2O$ (44 g) was dissolved in 400 ml of boiling water in the presence of $SnCl_2$ (2 g) in a $N_2$ atmosphere. The solution was filtered while hot to remove any undissolved material. To the dark green filtrate was added 200 ml of 52% aqueous HF, which was placed in a plastic beaker. Gamma-alumina (200 g) was gradually added to the dark green solution in such a way to maintain the temperature at 45°C. At the end of addition, the mixture turned to a light green solution, which was left at room temperature for 60 hours. The mixture was then filtered and acetone washed, until the filtrate was acid-free. The collected solid was air dried, followed by gradual heating at 100°C for two hours, 150°C for two hours, and finally at 175°C for 18 hours. The solid obtained was ground with a mortar and pestle and sieved. The 50-100 mesh particles (48g) were collected and loaded into the same reactor utilized in Example 1. The reactor was placed in a tube furnace and gradually heated electrically. The catalyst was activated for 18 hours at 650°C with air (20 cm³/min.), followed by HF (0.03 g/min.) and $N_2$ (20 cm³/min.) for approximately 20 hours. A total of 35 g of HF was fed to the reactor. A mixture of 75% $N_2$ and 25% 1,1,1-trifluoroethane, by volume, was fed to the reactor at 400°C (Comp. Ex. 11), 450°C (Comp. Ex. 12), and 550°C (Comp. Ex. 13). The conversion of 1,1,1-trifluoroethane reached a maximum of 6% at 400°C over 6.5 hours, increasing to 22% and 7.5 hours at 450°C. A fast deactivation from 37% to 22% was observed in 20 hours at 550°C. The results are tabulated in Table 1.

**Example 14**

The procedure of Comparative Example 13 (550°C) was repeated except that the reactor feed was a mixture of 75% $N_2$, 1.6% $O_2$ and 24.4% 1,1,1-trifluoroethane, by volume. Catalyst lifetime increased dramatically from 20 hours to 104 hours, due to the inclusion of the $O_2$ co-reactor gas in the reactor feed. Conversion of 1,1,1-trifluoroethane gradually improved from an initial value of 31% to a final value of 45% over 104 hours of continuous reactor operation. There was no evidence of catalytic deactivation. Mass balance exceeded 95%, and selectivity for vinylidene fluoride was 100%.

It is apparent from a comparison of Example 14 with Comparative Example 13, that the inclusion of the co-reactant gas in the dehydrofluorination process increases catalyst longevity. A catalyst lifetime of only 20 hours for Example 13 was increased to 104 hours by adding 1.6% $O_2$ (Example 14).

**Examples 15-16**

An 18 wt.% $CrF_3/AlF_3$ catalyst was prepared as follows. $CrF_3 \cdot 3H_2O$ (88 g) was dissolved in 500 ml of water using a Soxhlet extractor. The dark green chromium solution was added to 500 ml of 52 wt.% aqueous HF. Gamma-alumina (E & M Chemicals, activated powder, 200 g) was gradually added to the dark green solution at 40-45°C over 6 hours. The resulting pale green suspension was left covered at room temperature for 60 hours. A white/greenish solid precipitate formed, which was filtered then washed with acetone until the filtrate was acid-free. After air drying, the large granules from the precipitate were heat-dried at 100°C for two hours, followed by further heating at 150°C for another two hours, and heating at 175°C for 18 hours. The solid was ground using a mortar and pestle, and sieved. Then, 38 g of the 80-100 mesh sieved catalyst was loaded into the same reactor used in Example 1. The catalyst was activated with 20 cm³/min. of air for 18 hours, followed by treatment with HF (0.03 g/min.) and $N_2$ (20 cm³/min.) for 18 hours. A mixture of $CO_2$ (45 cm³/min.) and 1,1,1-trifluoroethane (15 cm³/min.) was fed to the reactor at 500°C (Example 15). Conversion of 1,1,1-trifluoroethane dropped from an initial rate of 33% to 20% over 24 hours. At 550°C (Example 16) conversion dropped from 42% to 22% over 21 hours. In both cases, selectivity for vinylidene fluoride was 100%.

**Example 17**

The procedure of Example 16 was repeated except that the reaction feed was a mixture of 74% $N_2$, 1.6% $O_2$ and 24.4% 1,1,1-trifluoroethane, by volume. An initial 1,1,1-trifluoroethane conversion rate of 18% increased to 27% over 20 hours, with 100% selectivity for vinylidene fluoride.

The results from Examples 1-17 are summarized in Table 1:

TABLE 1

| Catalyst | | Example | Reactor Gas Feed (vol%) | | | | T° C | %CH₃CF₃ Conversion | | Catalyst Lifetime (Hours) |
|---|---|---|---|---|---|---|---|---|---|---|
| | | | $N_2$ | $O_2$ | $CO_2$ | $CH_3CF_3$ | | Init. | Final | |
| Gamma-Fluorided alumina | | 1 | 75 | 1.6 | − | 23.4 | 500 | 18 | | 20 |
| " | | 2 | 75 | 1.6 | − | 23.4 | 550 | 42 | 44 | 7.5 |
| " | | 3 | − | 6.25 | − | 93.75 | 550 | 45 | 40 | 240 |
| 18 wt.% CrF₃/Al₂O₃ | | 4 | − | − | 75 | 25 | 400 | 27 | 18 | 13 |
| " | | 5 | − | − | 75 | 25 | 500 | 32 | 42 | 45 |
| " | | 6 | − | − | 75 | 25 | 550 | 65 | 56 | 33 |
| 8.5 wt.% CrF₃/Al₂O₃ | | 7 | − | − | 75 | 25 | 500 | 38.5 | 42.7 | 35 |
| " | | 8 | − | − | 75 | 25 | 550 | 52 | 40 | 20 |
| " | | 9 | 74 | 1.6 | − | 24.4 | 500 | 37.2 | 38.7 | 48 |
| " | | 10 | 74 | 1.6 | − | 24.4 | 550 | 48 | 56.6 | 24 |
| 8.5 wt.% CrF₃/AlF₃ | Comp. | 11 | 75 | − | − | 25 | 400 | 0 | 6 | 6.5 |
| " | Comp. | 12 | 75 | − | − | 25 | 450 | 7.5 | 22 | 7.5 |
| " | Comp. | 13 | 75 | − | − | 25 | 550 | 37 | 22 | 20 |
| " | | 14 | 75 | 1.6 | − | 24.4 | 550 | 31 | 45 | 104 |
| 18 wt.% CrF₃/AlF₃ | | 15 | 75 | 25 | 75 | 25 | 500 | 33 | 20 | 24 |
| " | | 16 | 75 | 25 | 75 | 25 | 550 | 42 | 22 | 21 |
| " | | 17 | 74 | 1.6 | − | 24.4 | 550 | 18 | 27 | 20 |

## Example 18

Gamma-alumina obtained from Harshaw/Filtrol (15 g) was loaded into the same reactor utilized in Example 1. Air at 650°C at a flow rate of 10 cm³/min. was flowed over the catalyst for 0 (i.e., no air activation), 8 and 24 hours. Thereafter, the catalyst was activated using hydrogen fluoride (0.05 g/min.) for six hours, with nitrogen (20 cm³/min.) as a carrier gas. A feed gas mixture of 93.2 mol% 1,1,1-trifluoroethane and 6.8 mol% $O_2$, by volume, was contacted with the catalyst for 98.8 seconds. The results are set forth in Table 2.

TABLE 2

| Catalyst Activation Air Flow (Hrs.) | % CH₃CF₃ Conversion |
|---|---|
| 0 | 33 |
| 8 | 41 |
| 24 | 50 |

It is clear from the data of Example 18 that the extent of reactant conversion is favored by a longer duration of catalyst air activation. Thus, when the catalyst was activated for 24 hours, 1,1,1-trifluoroethane conversion was 50%, versus 41% for 8 hours activation, and 33% in the absence of air activation.

**Example 19**

Harshaw $\gamma$-alumina (16 g) was loaded into the reactor described in Example 1. The catalyst was activated first with 20 cm$^3$/min. air at 650°C for 24 hours, followed by HF (0.07 g/min. and N$_2$ (20 cm$^3$/min.) for 5 hours at the same temperature. The temperature was lowered to 550°C, and a mixture of 1,1,1-trifluoroethane and air (72.5%/27.5%, by volume) was continuously flowed over the activated catalyst bed. The initial conversion of 1,1,1-trifluoroethane was 54.2%. After approximately 600 hours of continuous operation, the conversion dropped only slightly, to 52.2%.

**Example 20**

$\gamma$-alumina (18.1 grams) obtained from United Catalyst was placed in the same reactor utilized in Example 1. The reactor was gradually heated to 650°C with the continuous feed of air from a valve at the reactor top at the rate of 20 cm$^3$/min. The temperature was maintained at 650°C with the same air flow rate for 18 hours. The catalyst was thereafter activated at 550°C with hydrogen fluoride fed at the rate of 0.06 g/min. for 6 hours from a valve at the reactor top, with nitrogen as a carrier gas at the rate of 20 cm$^3$/min., to form fluorided $\gamma$-alumina. A mixture of 86.8% 1,1-difluoroethane, 3.2% O$_2$ and 10.0% N$_2$, by volume, was fed into the reactor and over the catalyst at 250°C. After 17 seconds contact time, 22.5% conversion of 1,1-difluoroethane to vinyl fluoride was measured, with 100% selectivity for vinyl fluoride.

**Example 21**

The procedure of Example 20 was repeated except that the reaction temperature was increased to 300°C and the reactor feed mixture was adjusted to 83.1% 1,1-difluoroethane, 4.27% O$_2$ and 12.63% N$_2$, by volume. The conversion of 1,1-difluoroethane was observed to increase to 53.7%, with selectivity for vinyl fluoride being 99.8%. The only other product was vinylidene fluoride, 0.2%.

**Examples 22-24**

The procedure of Example 21 was repeated, except the temperature was increased to 349°C, 399°C and 450°C. The data is summarized in Table 3.

TABLE 3

| 1,1-Difluoroethane Conversion to Vinyl Fluoride and Vinylidene Fluoride with Fluorided $\gamma$-alumina Catalyst | | | | | |
|---|---|---|---|---|---|
| Example | 20 | 21 | 22 | 23 | 24 |
| Reaction Temp. (°C) | 250 | 300 | 349 | 399 | 450 |
| Contact Time (sec.) | 17.0 | 19.6 | 18.6 | 17.2 | 15.0 |
| 1,1-Difluoroethane feed, (vol.%) | 86.8 | 83.1 | 82.8 | 82.8 | 83.9 |
| O$_2$ (vol.), per 100 volumes 1,1-difluoroethane | 3.19 | 4.27 | 4.26 | 4.32 | 4.04 |
| 1,1-Difluoroethane Conversion (mole %) | 22.5 | 53.7 | 77.9 | 93.1 | 96.5 |
| Vinylidene Fluoride Product (mole %) | 0 | 0.2 | 1.3 | 6.2 | 15.8 |
| Vinyl Fluoride Product (mole %) | 100.0 | 99.8 | 98.7 | 93.8 | 84.2 |

It may be appreciated from a consideration of Table 3 that conversion of 1,1-difluoroethane increased progressively from 22.5% at 250°C to 96.5% at 450°C. Meanwhile, vinylidene fluoride selectivity gradually increased from 0% at 250°C to 16% at 450°C.

The present invention may be embodied in other specific forms without departing from the spirit or essential attributes thereof and, accordingly, reference should be made to the appended claims, rather than to the foregoing specification, as indicating the scope of the invention.

## Claims

1. A process for dehydrofluorinating a fluorinated alkane of the formula $R_1R_2CHCFR_3R_4$ wherein each of $R_1$, $R_2$, $R_3$ and $R_4$ are independently selected from the group of hydrogen, fluorine, methyl, ethyl, propyl, t-butyl and phenyl comprising flowing said fluorinated alkane and a co-reactant gas containing oxygen, carbon dioxide or mixtures thereof over a catalyst bed which includes fluorided $\gamma$-alumina at a reaction temperature of from 200°C to 700°C, said catalyst bed having been prepared by the steps of
   (a) flowing air over a catalyst bed including $\gamma$-alumina for a contact time of from 5 seconds to 150 seconds, at a temperature of from 600°C to 750°C, and
   (b) thereafter flowing hydrogen fluoride over the catalyst bed at elevated temperature to convert the $\gamma$-alumina to $\gamma$-fluorided alumina.

2. A process according to claim 1 wherein the co-reactant gas comprises oxygen in the amount of from 2 to 10 volumes, per 100 volumes of the fluorinated alkane.

3. A process according to claim 2 wherein the co-reactant gas comprises oxygen in the amount of from 4 to 6 volumes, per 100 volumes of the fluorinated alkane.

4. A process according to claim 1 wherein the co-reactant gas comprises carbon dioxide, in the amount of from 50 to 400 volumes, per 100 volumes of the fluorinated alkane.

5. A process according to claim 1 wherein the co-reactant gas comprises air.

6. A process according to claim 1 wherein the co-reactant gas comprises substantially pure oxygen.

7. A process according to claim 1 wherein the fluorinated alkane and co-reactant gas are mixed with a carrier gas.

8. A process according to claim 1 wherein the catalyst is $\gamma$-fluorided alumina.

9. A process according to claim 1 wherein the catalyst includes at least one salt or oxide of chromium (III) or iron (III).

10. A process according to claim 9 wherein the catalyst includes $CrF_3$.

11. A process according to claim 1 wherein the reaction temperature is from 400°C to 700°C.

12. A process according to claim 11 where the reaction temperature is from 500°C to 650°C.

13. A process according to claim 1 for producing a difluoroalkene from a trifluoroalkane.

14. A process according to claim 13 for producing a 1,1-difluoroalkene from a 1,1,1-trifluoroalkane.

15. A process according to claim 14 for producing vinylidene fluoride from 1,1,1-trifluoroethane.

16. A process according to claim 1 for producing a fluoroalkene from a difluoroalkane.

17. A process according to claim 1 for producing a trifluoroalkene from a tetrafluoroalkane.

18. A process according to claim 17 for producing 1,1,2-trifluoroethylene from 1,1,1,2-tetrafluoroethane.

19. A process according to claim 1 wherein step (a) of the catalyst preparation comprises continuously flowing air over the catalyst bed for a contact time of from 20 seconds to 120 seconds, at a temperature of from 650°C to 700°C.

20. A process according to claim 1 wherein the hydrogen fluoride is flowed over the catalyst bed in step (b) of the catalyst preparation at a temperature of from 600°C to 700°C.

**21.** A process according to claim 1 wherein a carrier gas is combined with the hydrogen fluoride in step (b) of the catalyst preparation in an amount of from 100 to 500 volumes, per 100 volumes of the hydrogen fluoride.

**22.** A process for producing a 1,1-difluoroalkene comprising continuously flowing a gaseous 1,1,1-trifluoroalkane of the formula $R_1CH_2$-$CF_3$ wherein $R_1$ is selected from the group of hydrogen, fluorine, methyl, ethyl, propyl, t-butyl and phenyl, and a co-reactant gas containing oxygen, carbon dioxide or mixtures thereof, over a catalyst bed which includes $\gamma$-fluorided alumina, at a reaction temperature of from 400°C to 700°C to form a 1,1-difluoroalkene of the formula $R_1CH$=$CF_2$, said catalyst bed having been prepared by the steps of
   (a) flowing air over a catalyst bed including $\gamma$-alumina for a contact time of from 5 seconds to 150 seconds, at a temperature of from 600°C to 750°C, and
   (b) thereafter flowing hydrogen fluoride over the catalyst bed at elevated temperature to convert the $\gamma$-alumina to $\gamma$-fluoride alumina.

**23.** A process according to claim 22 wherein hydrogen fluoride is flowed over the catalyst bed in step (b) of the catalyst preparation at a temperature of from 600°C to 700°C.

**24.** A process according to claim 22 wherein the co-reactant gas comprises oxygen.

**25.** A process according to claim 24 wherein the co-reactant gas comprises oxygen in the amount of from 2 to 10 volumes, per 100 volumes of the 1,1,1-trifluoroalkane.

**26.** A process according to claim 25 wherein the co-reactant gas comprises oxygen in the amount of from 4 to 6 volumes, per 100 volumes of the 1,1,1-trifluoroalkane.

**27.** A process according to claim 25 wherein the co-reactant gas is air.

**28.** A process according to claim 25 wherein the co-reactant gas is substantially pure oxygen.

**29.** A process according to claim 22 wherein the catalyst is $\gamma$-fluorided alumina.

**30.** A process according to claim 22 wherein the catalyst includes at least one salt or oxide of chromium (III) or iron (III).

**31.** A process according to claim 22 for producing vinylidene fluoride from 1,1,1-trifluoroethane.

**32.** A process according to claim 22 wherein step (a) of the catalyst preparation comprises continuously flowing air over the catalyst bed for a contact time from 20 seconds to 120 seconds, at a temperature of from 650°C to 700°C.

**33.** A process for producing vinylidene fluoride from 1,1,1-trifluoroethane comprising continuously feeding gaseous 1,1,1-trifluoroethane and from about 2 to about 10 volumes of oxygen, per 100 volumes of the 1,1,1-trifluoroethane, over a catalyst bed comprising fluorided $\gamma$-alumina at a reaction temperature of from 400°C to 700°C to form vinylidene fluoride, said catalyst bed having been prepared by the steps of
   (a) flowing air over a $\gamma$-alumina bed for a contact time of from 5 second to 150 seconds, at a temperature of from 600°C to 700°C; and
   (b) thereafter flowing gaseous hydrogen fluoride over the catalyst bed at a temperature of from 500°C to 600°C to convert the $\gamma$-alumina to fluorided $\gamma$-alumina.

**34.** A process according to claim 33 wherein the amount of oxygen is about 4 to 6 volumes, per 100 volumes of the 1,1,1-trifluoroethane.

**35.** A process according to claim 33 wherein the reaction temperature is from 500°C to 650°C.

**36.** A process for producing vinylidene fluoride comprising

12

(a) flowing 1,1-difluoroethane and a co-reactant gas containing oxygen, carbon dioxide or mixtures thereof over a catalyst bed which includes fluorided γ-alumina at a reaction temperature of from 300°C to 700°C, to obtain a product mixture comprising vinyl fluoride and vinylidene fluoride, said catalyst bed having been prepared by the steps of

(i) flowing air over a catalyst bed including γ-alumina for a contact time of from 5 seconds to 150 seconds, at a temperature of from 600°C to 750°C, and

(ii) thereafter flowing hydrogen fluoride over the catalyst bed at elevated temperature to convert the γ-alumina to γ-fluorided alumina,

(b) recovering vinylidene fluoride from said mixture.

37. A process according to claim 36 wherein the reaction temperature is from 400°C to 550°C.

38. A process according to claim 36 wherein the co-reactant gas comprises oxygen, carbon dioxide or mixture thereof.

39. A process according to claim 38 wherein the co-reactant gas is air.

40. A process according to claim 38 wherein the co-reactant gas is oxygen.

41. A process according to claim 38 wherein the co-reactant gas is carbon dioxide.

42. A process according to claim 36 wherein the molar ratio of 1,1-difluoroethane to co-reactant gas is from 0.04:1 to 10:1.

43. A process according to claim 42 wherein the molar ratio of 1,1-difluoroethane to co-reactant gas is from 0.5:1 to 4:1.

44. A process according to claim 36 wherein the contact time is from 1 second to 60 seconds.

45. A process according to claim 44 wherein the contact time is from 5 seconds to 20 seconds.

46. A process according to claim 36 wherein the catalyst is fluorided γ-alumina.

47. A process according to claim 42 wherein the contact time is from 1 second to 60 seconds.

48. A process according to claim 47 wherein the reaction temperature is from 400°C to 550°C.

49. A process according to claim 47 wherein the molar ratio of 1,1-difluoroethane to oxidizing gas is from 0.5:1 to 10:1.

50. A process according to claim 48 wherein the contact time is from 5 seconds to 20 seconds.

**Patentansprüche**

1. Verfahren zur Dehydrofluorierung eines fluorierten Alkans der Formel $R_1 R_2 CHCFR_3 R_4$, worin jeder der Substituenten $R_1$, $R_2$, $R_3$ und $R_4$ unabhängig aus der Gruppe Wasserstoff, Fluor, Methyl, Ethyl, Propyl, t-Butyl und Phenyl ausgewählt wird, dadurch **gekennzeichnet,** daß das fluorierte Alkan und ein Coreaktionsgas, welches Sauerstoff, Kohlendioxid oder Gemische davon enthält, über eine Katalysatorschicht, welche fluoridiertes γ-Aluminiumoxid enthält, bei einer Reaktionstemperatur von 200°C bis 700°C geleitet werden, wobei die Katalysatorschicht nach den folgenden Stufen hergestellt worden ist

(a) Leiten von Luft über eine Katalysatorschicht, welche γ-Aluminiumoxid umfaßt, für eine Kontaktzeit von 5 Sekunden bis 150 Sekunden, bei einer Temperatur von 600°C bis 750°C, und

(b) anschließendem Leiten von Fluorwasserstoff über die Katalysatorschicht bei erhöhter Temperatur, um das γ-Aluminiumoxid in γ-fluoridiertes Aluminiumoxid zu überführen.

2. Verfahren nach Anspruch 1, dadurch **gekennzeichnet,** daß das Coreaktionsgas Sauerstoff in einer Menge von 2 bis 10 Volumen, pro 100 Volumen fluoriertem Alkan, enthält.

**3.** Verfahren nach Anspruch 2, dadurch **gekennzeichnet,** daß das Coreaktionsgas Sauerstoff in einer Menge von 4 bis 6 Volumen, pro 100 Volumen fluoriertem Alkan, enthält.

**4.** Verfahren nach Anspruch 1, dadurch **gekennzeichnet,** daß das Coreaktionsgas Kohlendioxid in einer Menge von 50 bis 400 Volumen, pro 100 Volumen fluoriertem Alkan, enthält.

**5.** Verfahren nach Anspruch 1, dadurch **gekennzeichnet,** daß das Coreaktionsgas Luft enthält.

**6.** Verfahren nach Anspruch 1, dadurch **gekennzeichnet,** daß das Coreaktionsgas im wesentlichen reinen Sauerstoff enthält.

**7.** Verfahren nach Anspruch 1, dadurch **gekennzeichnet,** daß das fluorierte Alkan und das Coreaktionsgas mit einem Trägergas vermischt werden.

**8.** Verfahren nach Anspruch 1, dadurch **gekennzeichnet,** daß der Katalysator $\gamma$-fluoridiertes Aluminiumoxid ist.

**9.** Verfahren nach Anspruch 1, dadurch **gekennzeichnet,** daß der Katalysator mindestens ein Salz oder Oxid aus Chrom(III) oder Eisen(III) enthält.

**10.** Verfahren nach Anspruch 9, dadurch **gekennzeichnet,** daß der Katalysator $CrF_3$ enthält.

**11.** Verfahren nach Anspruch 1, dadurch **gekennzeichnet,** daß die Reaktionstemperatur 400°C bis 700°C beträgt.

**12.** Verfahren nach Anspruch 11, dadurch **gekennzeichnet,** daß die Reaktionstemperatur 500°C bis 650°C beträgt.

**13.** Verfahren nach Anspruch 1, dadurch **gekennzeichnet,** daß ein Difluoralken aus einem Trifluoralkan hergestellt wird.

**14.** Verfahren nach Anspruch 13, dadurch **gekennzeichnet,** daß 1,1-Difluoralken aus 1,1,1-Trifluoralkan hergestellt wird.

**15.** Verfahren nach Anspruch 14, dadurch **gekennzeichnet,** daß Vinylidenfluorid aus 1,1,1-Trifluorethan hergestellt wird.

**16.** Verfahren nach Anspruch 1, dadurch **gekennzeichnet,** daß Fluoralken aus Difluoralkan hergestellt wird.

**17.** Verfahren nach Anspruch 1, dadurch **gekennzeichnet,** daß Trifluoralken aus Tetrafluoralkan hergestellt wird.

**18.** Verfahren nach Anspruch 17, dadurch **gekennzeichnet,** daß 1,1,2-Trifluorethylen aus 1,1,1,2-Tetrafluorethan hergestellt wird.

**19.** Verfahren nach Anspruch 1, dadurch **gekennzeichnet,** daß die Stufe (a) der Katalysatorherstellung das kontinuierliche Leiten von Luft über die Katalysatorschicht während einer Kontaktzeit von 20 Sekunden bis 120 Sekunden, bei einer Temperatur von 650°C bis 700°C, umfaßt.

**20.** Verfahren nach Anspruch 1, dadurch **gekennzeichnet,** daß der Fluorwasserstoff über die Katalysatorschicht bei der Stufe (b) der Katalysatorherstellung, bei einer Temperatur von 600°C bis 700°C, geleitet wird.

**21.** Verfahren nach Anspruch 1, dadurch **gekennzeichnet,** daß ein Trägergas mit dem Fluorwasserstoff bei der Stufe (b) der Katalysatorherstellung in einer Menge von 100 bis 500 Volumen, pro 100 Volumen Fluorwasserstoff, kombiniert wird.

**22.** Verfahren zur Herstellung eines 1,1-Difluoralkens, dadurch **gekennzeichnet,** daß kontinuierlich ein gasförmiges 1,1,1-Trifluoralkan der Formel $R_1CH_2$-$CF_3$, worin $R_1$ ausgewählt wird aus der Gruppe Wasserstoff, Fluor, Methyl, Ethyl, Propyl, t-Butyl und Phenyl, und ein Coreaktionsgas, welches Sauerstoff, Kohlendioxid oder Gemische davon enthält, über eine Katalysatorschicht, welche $\gamma$-fluoridiertes Aluminiumoxid umfaßt, bei einer Reaktionstemperatur von 400°C bis 700°C, unter Bildung von 1,1-Difluoralken der Formel $R_1CH = CF_2$ geleitet wird, wobei die Katalysatorschicht durch die folgenden Stufen hergestellt worden ist

(a) Leiten von Luft über eine Katalysatorschicht, welche $\gamma$-Aluminiumoxid umfaßt, während einer Kontaktzeit von 5 Sekunden bis 150 Sekunden, bei einer Temperatur von 600°C bis 750°C, und

(b) anschließendes Leiten von Fluorwasserstoff über die Katalysatorschicht bei erhöhter Temperatur, um das $\gamma$-Aluminiumoxid in $\gamma$-Fluoridaluminiumoxid zu überführen.

**23.** Verfahren nach Anspruch 22, dadurch **gekennzeichnet,** daß der Fluorwasserstoff über die Katalysatorschicht bei der Stufe (b) der Katalysatorherstellung bei einer Temperatur von 600°C bis 700°C geleitet wird.

**24.** Verfahren nach Anspruch 22, dadurch **gekennzeichnet,** daß das Coreaktionsgas Sauerstoff enthält.

**25.** Verfahren nach Anspruch 24, dadurch **gekennzeichnet,** daß das Coreaktionsgas Sauerstoff in einer Menge von 2 bis 10 Volumen, pro 100 Volumen 1,1,1-Trifluoralkan, enthält.

**26.** Verfahren nach Anspruch 25, dadurch **gekennzeichnet,** daß das Coreaktionsgas Sauerstoff in einer Menge von 4 bis 6 Volumen, pro 100 Volumen 1,1,1-Trifluoralkan, enthält.

**27.** Verfahren nach Anspruch 25, dadurch **gekennzeichnet,** daß das Coreaktionsgas Luft ist.

**28.** Verfahren nach Anspruch 25, dadurch **gekennzeichnet,** daß das Coreaktionsgas im wesentlichen reiner Sauerstoff ist.

**29.** Verfahren nach Anspruch 22, dadurch **gekennzeichnet,** daß der Katalysator $\gamma$-fluoridiertes Aluminiumoxid ist.

**30.** Verfahren nach Anspruch 22, dadurch **gekennzeichnet,** daß der Katalysator mindestens ein Salz oder Oxid aus Chrom(III) oder Eisen(III) enthält.

**31.** Verfahren nach Anspruch 22, dadurch **gekennzeichnet,** daß Vinylidenfluorid aus 1,1,1-Trifluorethan hergestellt wird.

**32.** Verfahren nach Anspruch 22, dadurch **gekennzeichnet,** daß die Stufe (a) der Katalysatorherstellung kontinuierliches Strömen von Luft über die Katalysatorschicht während einer Kontaktzeit von 20 Sekunden bis 120 Sekunden bei einer Temperatur von 650°C bis 700°C umfaßt.

**33.** Verfahren zur Herstellung von Vinylidenfluorid aus 1,1,1-Trifluorethan, dadurch **gekennzeichnet,** daß gasförmiges 1,1,1-Trifluorethan und von etwa 2 bis etwa 10 Volumen Sauerstoff, pro 100 Volumen 1,1,1-Trifluorethan, über eine Katalysatorschicht, welche fluoridiertes $\gamma$-Aluminiumoxid enthält, bei einer Reaktionstemperatur von 400°C bis 700°C, unter Bildung von Vinylidenfluorid kontinuierlich geleitet werden, wobei die Katalysatorschicht gemäß den folgenden Stufen hergestellt worden ist:

(a) Leiten von Luft über eine $\gamma$-Aluminiumoxidschicht während einer Kontaktzeit von 5 Sekunden bis 150 Sekunden bei einer Temperatur von 600°C bis 700°C; und

(b) anschließendem Leiten von gasförmigem Fluorwasserstoff über die Katalysatorschicht bei einer Temperatur von 500°C bis 600°C, um das $\gamma$-Aluminiumoxid in fluoridiertes $\gamma$-Aluminiumoxid zu überführen.

**34.** Verfahren nach Anspruch 33, dadurch **gekennzeichnet,** daß die Menge an Sauerstoff 4 bis 6 Volumen, pro 100 Volumen 1,1,1-Trifluorethan, beträgt.

**35.** Verfahren nach Anspruch 33, dadurch **gekennzeichnet,** daß die Reaktionstemperatur von 500°C bis 650°C beträgt.

EP 0 406 748 B1

**36.** Verfahren zur Herstellung von Vinylidenfluorid, dadurch **gekennzeichnet, daß**

(a) 1,1,-Difluorethan und ein Coreaktionsgas, welches Sauerstoff, Kohlendioxid oder Gemische davon enthält, über eine Katalysatorschicht, welche fluoridiertes $\gamma$-Aluminiumoxid enthält, bei einer Reaktionstemperatur von 300°C bis 700°C geleitet werden, wobei ein Produktgemisch erhalten wird, welches Vinylfluorid und Vinylidenfluorid enthält, wobei die Katalysatorschicht gemäß den folgenden Stufen hergestellt worden ist

(i) Leiten von Luft über eine Katalysatorschicht, welche $\gamma$-Aluminiumoxid umfaßt, während einer Kontaktzeit von 5 Sekunden bis 150 Sekunden bei einer Temperatur von 600°C bis 750°C, und

(ii) anschließendes Leiten von Fluorwasserstoff über die Katalysatorschicht bei einer erhöhten Temperatur, um das $\gamma$-Aluminiumoxid in $\gamma$-fluoridiertes Aluminiumoxid zu überführen, und

(b) Gewinnung von Vinylidenfluorid aus dem Gemisch.

**37.** Verfahren nach Anspruch 36, dadurch **gekennzeichnet,** daß die Reaktionstemperatur von 400°C bis 550°C beträgt.

**38.** Verfahren nach Anspruch 36, dadurch **gekennzeichnet,** daß das Coreaktionsgas Sauerstoff, Kohlendioxid oder ein Gemisch davon enthält.

**39.** Verfahren nach Anspruch 38, dadurch **gekennzeichnet,** daß das Coreaktionsgas Luft ist.

**40.** Verfahren nach Anspruch 38, dadurch **gekennzeichnet,** daß das Coreaktionsgas Sauerstoff ist.

**41.** Verfahren nach Anspruch 38, dadurch **gekennzeichnet,** daß das Coreaktionsgas Kohlendioxid ist.

**42.** Verfahren nach Anspruch 36, dadurch **gekennzeichnet,** daß das Molverhältnis von 1,1-Difluorethan zu Coreaktionsgas von 0,04:1 bis 10:1 beträgt.

**43.** Verfahren nach Anspruch 42, dadurch **gekennzeichnet,** daß das Molverhältnis von 1,1-Difluorethan zu Coreaktionsgas von 0,5:1 bis 4:1 beträgt.

**44.** Verfahren nach Anspruch 36, dadurch **gekennzeichnet,** daß die Kontaktzeit von 1 Sekunde bis 60 Sekunden beträgt.

**45.** Verfahren nach Anspruch 44, dadurch **gekennzeichnet,** daß die Kontaktzeit von 5 Sekunden bis 20 Sekunden beträgt.

**46.** Verfahren nach Anspruch 36, dadurch **gekennzeichnet,** daß der Katalysator fluoridiertes $\gamma$-Aluminiumoxid ist.

**47.** Verfahren nach Anspruch 42, dadurch **gekennzeichnet,** daß die Kontaktzeit von 1 Sekunde bis 60 Sekunden beträgt.

**48.** Verfahren nach Anspruch 47, dadurch **gekennzeichnet,** daß die Reaktionstemperatur von 400°C bis 550°C beträgt.

**49.** Verfahren nach Anspruch 47, dadurch **gekennzeichnet,** daß das Molverhältnis von 1,1-Difluorethan zu Oxidationsgas von 0,5:1 bis 10:1 beträgt.

**50.** Verfahren nach Anspruch 48, dadurch **gekennzeichnet,** daß die Kontaktzeit von 5 Sekunden bis 20 Sekunden beträgt.

**Revendications**

**1.** Procédé de déshydrofluoration d'un alcane fluoré de formule $R_1 R_2 CHCFR_3 R_4$, dans laquelle chacun de $R_1$, $R_2$, $R_3$ et $R_4$ est choisi, indépendamment, dans le groupe comprenant l'hydrogène, le fluor, les restes méthyle, éthyle, propyle, tertio-butyle et phényle, comprenant le passage d'un courant dudit alcane fluoré et d'un gaz co-réactionnel contenant de l'oxygène, de l'anhydride carbonique ou des mélanges de ces gaz sur un lit de catalyseur qui contient de la $\gamma$-alumine fluorée, à une température

16

de réaction de 200°C à 700°C, ce lit de catalyseur ayant été préparé par des étapes consistant

(a) à faire passer de l'air sur un lit de catalyseur contenant de la γ-alumine pendant une durée de contact de 5 à 150 secondes à une température de 600 à 750°C, puis

(b) à faire passer du fluorure d'hydrogène sur le lit de catalyseur à une température élevée pour convertir la γ-alumine en γ-alumine fluorée.

2. Procédé suivant la revendication 1, dans lequel le gaz co-réactionnel comprend de l'oxygène en une quantité de 2 à 10 volumes pour 100 volumes de l'alcane fluoré.

3. Procédé suivant la revendication 2, dans lequel le gaz co-réactionnel comprend de l'oxygène en quantité de 4 à 6 volumes pour 100 volumes de l'alcane fluoré.

4. Procédé suivant la revendication 1, dans lequel le gaz co-réactionnel comprend de l'anhydride carbonique en quantité de 50 à 400 volumes pour 100 volumes de l'alcane fluoré.

5. Procédé suivant la revendication 1, dans lequel le gaz co-réactionnel comprend de l'air.

6. Procédé suivant la revendication 1, dans lequel le gaz co-réactionnel comprend de l'oxygène pratiquement pur.

7. Procédé suivant la revendication 1, dans lequel l'alcane fluoré et le gaz co-réactionnel sont mélangés avec un véhicule gazeux.

8. Procédé suivant la revendication 1, dans lequel le catalyseur est de la γ-alumine fluorée.

9. Procédé suivant la revendication 1, dans lequel le catalyseur contient au moins un sel ou oxyde de chrome (III) ou de fer (III).

10. Procédé suivant la revendication 9, dans lequel le catalyseur contient du $CrF_3$.

11. Procédé suivant la revendication 1, dans lequel la température de réaction va de 400°C à 700°C.

12. Procédé suivant la revendication 11, dans lequel la température de réaction va de 500°C à 650°C.

13. Procédé suivant la revendication 1, pour la production d'un alcène difluoré à partir d'un alcane trifluoré.

14. Procédé suivant la revendication 13, pour la production d'un alcène difluoré en position 1 à partir d'un alcane trifluoré en position 1.

15. Procédé suivant la revendication 14, pour la production de fluorure de vinylidène à partir de 1,1,1-trifluoréthane.

16. Procédé suivant la revendication 1, pour la production d'un alcène fluoré à partir d'un alcane difluoré.

17. Procédé suivant la revendication 1, pour la production d'un alcène trifluoré à partir d'un alcane tétrafluoré.

18. Procédé suivant la revendication 17, pour la production de 1,1,2-trifluoréthylène à partir de 1,1,1,2-tétrafluoréthane.

19. Procédé suivant la revendication 1, dans lequel l'étape (a) de préparation du catalyseur comprend le passage continu d'air sur le lit de catalyseur pendant une durée de contact allant de 20 à 120 secondes à une température de 650 à 700°C.

20. Procédé suivant la revendication 1, dans lequel on fait passer le fluorure d'hydrogène sur le lit de catalyseur dans l'étape (b) de préparation du catalyseur, à une température de 600 à 700°C.

**21.** Procédé suivant la revendication 1, dans lequel un véhicule gazeux est mélangé au fluorure d'hydrogène dans l'étape (b) de préparation du catalyseur en une quantité de 100 à 500 volumes pour 100 volumes du fluorure d'hydrogène.

**22.** Procédé de production d'un 1,1-difluoralcène, comprenant le passage continu d'un 1,1,1-trifluoralcane gazeux de formule $R_1CH_2$-$CF_3$ dans laquelle $R_1$ est choisi dans le groupe comprenant l'hydrogène, le fluor, les restes méthyle, éthyle, propyle, tertio-butyle et phényle, et un gaz co-réactionnel contenant de l'oxygène, de l'anhydride carbonique ou des mélanges de ces gaz, sur un lit de catalyseur qui contient de la $\gamma$-alumine fluorée, à une température de réaction de 400 à 700°C pour former un 1,1-difluoralcène de formule $R_1CH=CF_2$, ce lit de catalyseur ayant été préparé par les étapes consistant :
(a) à faire passer de l'air sur un lit de catalyseur contenant de l'alumine $\gamma$ pendant une durée de contact de 5 à 150 secondes à une température de 600 à 750°C, puis
(b) à faire passer du fluorure d'hydrogène sur le lit de catalyseur à une température élevée pour convertir la $\gamma$-alumine en $\gamma$-alumine fluorée.

**23.** Procédé suivant la revendication 22, dans lequel on fait passer le fluorure d'hydrogène sur le lit de catalyseur dans l'étape (b) de préparation du catalyseur à une température de 600 à 700°C.

**24.** Procédé suivant la revendication 22, dans lequel le gaz co-réactionnel comprend de l'oxygène.

**25.** Procédé suivant la revendication 24, dans lequel le gaz co-réactionnel comprend de l'oxygène en quantité de 2 à 10 volumes pour 100 volumes de 1,1,1-trifluoralcane.

**26.** Procédé suivant la revendication 25, dans lequel le gaz co-réactionnel comprend de l'oxygène en quantité de 4 à 6 volumes pour 100 volumes de 1,1,1-trifluoralcane.

**27.** Procédé suivant la revendication 25, dans lequel le gaz co-réactionnel est l'air.

**28.** Procédé suivant la revendication 25, dans lequel le gaz co-réactionnel est de l'oxygène pratiquement pur.

**29.** Procédé suivant la revendication 22, dans lequel le catalyseur est de la $\gamma$-alumine fluorée.

**30.** Procédé suivant la revendication 22, dans lequel le catalyseur contient au moins un sel ou oxyde de chrome (III) ou de fer (III).

**31.** Procédé suivant la revendication 22, pour la production de fluorure de vinylidène à partir de 1,1,1-trifluoréthane.

**32.** Procédé suivant la revendication 22, dans lequel l'étape (a) de préparation du catalyseur comprend le passage continu d'air sur le lit de catalyseur pendant une durée de contact de 20 à 120 secondes, à une température de 650 à 700°C.

**33.** Procédé de production de fluorure de vinylidène à partir de 1,1,1-trifluoréthane, comprenant le passage continu de 1,1,1-trifluoréthane gazeux et d'environ 2 à environ 10 volumes d'oxygène, pour 100 volumes du 1,1,1-trifluoréthane, sur un lit de catalyseur comprenant de la $\gamma$-alumine fluorée à une température de réaction de 400 à 700°C pour former du fluorure de vinylidène, ce lit de catalyseur ayant été préparé par les étapes consistant :
(a) à faire passer de l'air sur un lit de $\gamma$-alumine pendant une durée de contact de 5 à 150 secondes, à une température de 600 à 700°C ; puis
(b) à faire passer du fluorure d'hydrogène gazeux sur le lit de catalyseur à une température de 500 à 600°C pour convertir la $\gamma$-alumine en $\gamma$-alumine fluorée.

**34.** Procédé suivant la revendication 33, dans lequel la quantité d'oxygène va de 4 à 6 volumes pour 100 volumes du 1,1,1-trifluoréthane.

**35.** Procédé suivant la revendication 33, dans lequel la température de réaction va de 500 à 650°C.

**36.** Procédé de production de fluorure de vinylidène, qui comprend les étapes consistant

(a) à faire passer du 1,1-difluoréthane et un gaz co-réactionnel contenant de l'oxygène, de l'anhydride carbonique ou des mélanges de ces gaz sur un lit de catalyseur qui contient de la $\gamma$-alumine fluorée, à une température de réaction de 300 à 700°C, pour obtenir comme produit un mélange comprenant du fluorure de vinyle et du fluorure de vinylidène, le lit de catalyseur ayant été préparé par les étapes consistant

(i) à faire passer de l'air sur un lit de catalyseur contenant de la $\gamma$-alumine pendant une durée de contact de 5 à 150 secondes à une température de 600 à 750°C, puis

(ii) à faire passer du fluorure d'hydrogène sur le lit de catalyseur à température élevée pour convertir la $\gamma$-alumine en $\gamma$-alumine fluorée,

(b) à séparer le fluorure de vinylidène dudit mélange.

**37.** Procédé suivant la revendication 36, dans lequel la température de réaction va de 400 à 550°C.

**38.** Procédé suivant la revendication 36, dans lequel le gaz co-réactionnel comprend de l'oxygène, de l'anhydride carbonique ou un mélange de ces gaz.

**39.** Procédé suivant la revendication 38, dans lequel le gaz co-réactionnel est l'air.

**40.** Procédé suivant la revendication 38, dans lequel le gaz co-réactionnel est de l'oxygène.

**41.** Procédé suivant la revendication 38, dans lequel le gaz co-réactionnel est de l'anhydride carbonique.

**42.** Procédé suivant la revendication 36, dans lequel le rapport molaire du 1,1-difluoréthane au gaz co-réactionnel va de 0,04:1 à 10:1.

**43.** Procédé suivant la revendication 42, dans lequel le rapport molaire du 1,1-difluoréthane au gaz co-réactionnel est d'environ 0,5:1 à 4:1.

**44.** Procédé suivant la revendication 36, dans lequel la durée de contact va de 1 seconde à 60 secondes.

**45.** Procédé suivant la revendication 44, dans lequel la durée de contact va de 5 à 20 secondes.

**46.** Procédé suivant la revendication 36, dans lequel le catalyseur est de la $\gamma$-alumine fluorée.

**47.** Procédé suivant la revendication 42, dans lequel la durée de contact va de 1 seconde à 60 secondes.

**48.** Procédé suivant la revendication 47, dans lequel la température de réaction va de 400°C à 550°C.

**49.** Procédé suivant la revendication 47, dans lequel le rapport molaire du 1,1-difluoréthane au gaz oxydant va de 0,5:1 à 10:1.

**50.** Procédé suivant la revendication 48, dans lequel la durée de contact va de 5 à 20 secondes.